# EUROPEAN PATENT SPECIFICATION

(11) **EP 4 483 852 B1**
(45) Date of publication and mention of the grant of the patent: **06.05.2026**
(21) Application number: 23779993.7
(22) Date of filing: 22.03.2023
(51) Int. Cl.: A61F 13/15, A61F 13/53, A61F 13/532

(54) **METHOD FOR MANUFACTURING ABSORBENT BODY**
VERFAHREN ZUR HERSTELLUNG EINES SAUGFÄHIGEN KÖRPERS
PROCÉDÉ DE FABRICATION D'UN CORPS ABSORBANT

(30) Priority: 28.03.2022 JP 2022051266
(43) Date of publication of application: 01.01.2025
(73) Proprietor: Zuiko Corporation, Ibaraki-shi, Osaka 5670082 (JP)
(72) Inventor: UMEBAYASHI Toyoshi, Ibaraki-shi, Osaka 567-0082 (JP)
(74) Representative: Hasegawa, Kan
(86) International application number: PCT/JP2023/011341
(87) International publication number: WO 2023/189981

(56) References cited:
- WO-A1-2021/002095
- JP-A- 2013 255 818
- JP-A- 2019 058 739
- JP-A- H11 318 977

## Description

### TECHNICAL FIELD

The present invention relates to a method for manufacturing an absorbent body including an absorbent material.

### BACKGROUND ART

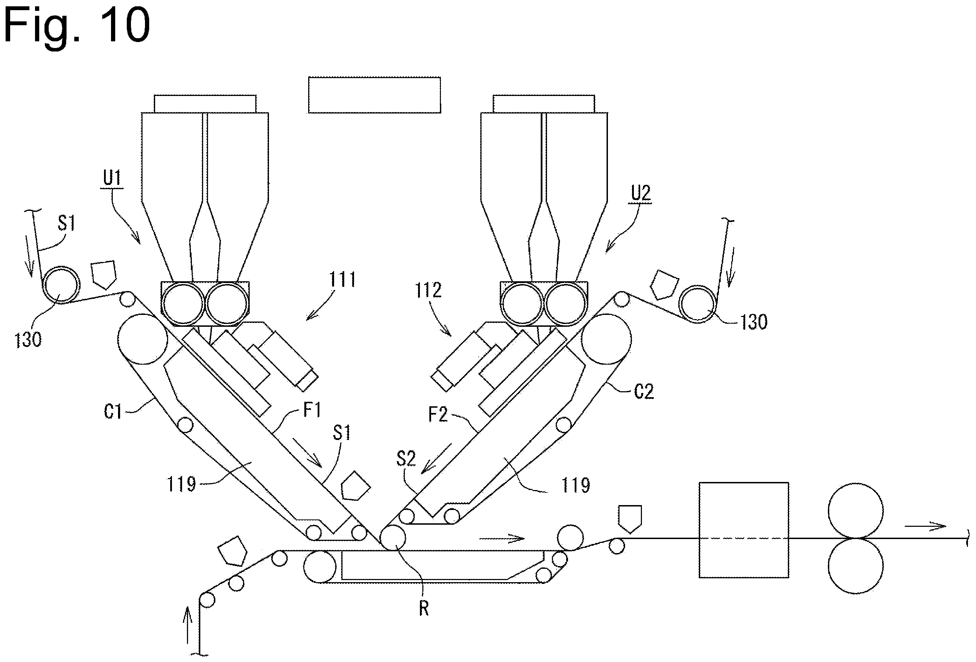

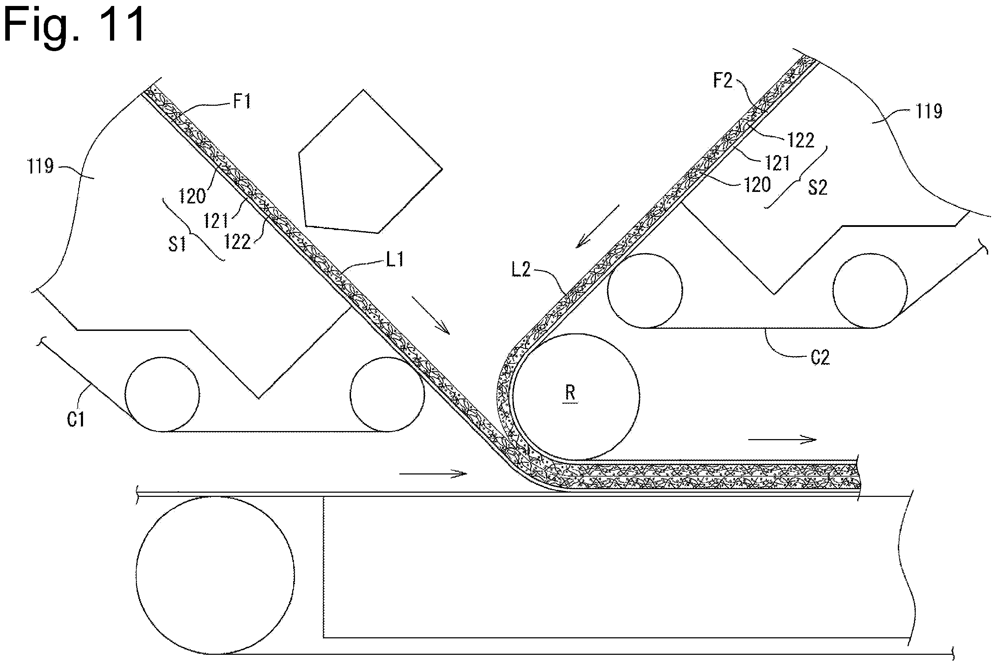

An absorbent body containing an absorbent material is used for diapers and sanitary napkins. For example, FIG. 10 is a schematic layout diagram of a manufacturing apparatus for manufacturing an absorbent body, and FIG. 11 is an enlarged cross-sectional view of a relevant part. As shown in FIGS. 10 and 11, this apparatus is provided with: two units U1 and U2 including conveyers C1 and C2 and sprayers 111 and 112, respectively; and a laminating roller R. The conveyers C1 and C2 each have a negative pressure case 119, and convey sheets S1 and S2 while sucking them. The sheets S1 and S2 each have a diffusion layer 121 and a short fiber layer 122, and the short fiber layer 122 is napped by a napping roller 130. The sprayers 111 and 112 spray a powder and granular material 120 as the absorbent material to the napped upper surfaces of the sheet S1 and S2 being conveyed along inclined surfaces F1 and F2. This forms absorbent layers L1 and L2 on the short fiber layers 122 of the sheets S1 and S2. The laminating roller R places the sheets S1 and S2 one on another so that the absorbent layers L1 and L2 face each other (for example, see WO 2021/015001 A1).

WO 2015/123087 A1 discloses to manufacture an absorbent body by placing one on another two sheets having an absorbent material transferred thereto.

WO 2021/002095 A1 discloses a method of producing absorbent body for absorbent article, wherein a base sheet having one main surface coated with an adhesive is supplied to a conveyance device and the base sheet is conveyed while the other main surface of the base sheet is suctioned. A first laminate (50p) is formed by overlapping the one main surface of the base sheet with the other main sheet of a fiber sheet having one main surface nap-raised; then, a granular absorbent material is scattered on the one main surface of the fiber sheet; the scattered absorbent material is infiltrated into the fiber sheet when the other main surface of the base sheet is suctioned; then, a second laminate is formed by overlapping the one main surface of the fiber sheet with the one main surface of a cover sheet coated with an adhesive; and then the resultant sheet is compressed in the thickness direction by means of a compression means and the coated adhesive spreads inside the fiber sheet.

### SUMMARY OF THE INVENTION

### PROBLEM TO BE SOLVED BY THE INVENTION

Merely placing two sheets one on another and disposing an absorbent material between the two sheets as described above limits variations of the absorbent body. For this reason, it is desired to further increase variations of the absorbent body.

In view of such circumstances, a problem to be solved by the present invention is to provide an absorbent body manufacturing method capable of increasing variations of the absorbent body.

### MEANS FOR SOLVING THE PROBLEM

To solve the above-mentioned problem, the present invention provides an absorbent body manufacturing method structured as follows:
An absorbent body manufacturing method is provided with: (i) a first step of forming a first absorbent sheet having a first absorbent material disposed in first regions of a first sheet; (ii) a second step of forming a second absorbent sheet having a second absorbent material disposed in second regions of a second sheet; (iii) a third step of forming a continuous body including the first absorbent sheet and the second absorbent sheet and in which the first regions and the second regions are adjacent to each other in plan view; and (iv) a fourth step of forming an absorbent body including the first absorbent material and the second absorbent material, by cutting the continuous body so that the first regions and the second regions adjacent to each other are included. At the first step, the first absorbent material is disposed in the first regions aligned in a direction in which the first sheet continues, in a plurality of rows with a spacing therebetween. At the second step, the second absorbent material is disposed in the second regions aligned in a direction in which the second sheet continues, with a spacing therebetween.

According to the above-described method, it is possible to change the characteristics of the absorbent body according to the pattern of the first and second regions and the kind and amount of the first and second absorbent materials disposed in the first and second regions, and a channel can be formed between a plurality of rows of the first regions aligned in the direction in which the first sheet continues. Consequently, variations of the absorbent body can be increased.

In an absorbent body manufacturing method of a preferred mode, at the third step, the continuous body is formed in which the first absorbent sheet and the second absorbent sheet overlap each other such that the first regions and the second regions are adjacent to each other in plan view.

In this case, the overlapping first and second absorbent sheets are simultaneously cut at the fourth step to form the absorbent body.

An absorbent body manufacturing method of another preferred mode is further provided with: (v) a fifth step of forming first pieces including the first regions, by cutting the first absorbent sheet after the first step and before the third step; and (vi) a sixth step of forming second pieces including the second region, by cutting the second absorbent sheet after the second step and before the third step. At the third step, the continuous body is formed in which the first pieces and the second pieces are aligned such that the first regions and the second regions are adjacent to each other in plan view.

In this case, since the continuous body includes the first and second absorbent sheets being cut and the first and second pieces that are the first and second absorbent sheets being cut do not overlap each other, the thickness of the absorbent body can be reduced, and the sheet materials used for the absorbent body can be reduced.

Preferably, at the first step, the first absorbent material is disposed on more than one of the first sheets. At the third step, the continuous body is formed in which more than one of the first sheets is arranged with a spacing therebetween.

In this case, a channel can be formed along the spacing between the adjoining first sheets. Moreover, because the plural first sheets are not restrained by one another, an absorbent body can be formed that easily follows the shape of the wearing part.

Preferably, at the first step, a first coating sheet is joined with the first sheet to form the first absorbent sheet having the first absorbent material disposed between the first sheet and the first coating sheet.

In this case, by adding the first coating sheet, variations of the absorbent body can be increased. Moreover, the movement of the first absorbent material toward the outside of the first regions can be suppressed, so that the quality of the absorbent body is stabilized.

Preferably, at the second step, the second coating sheet is joined with the second sheet to form the second absorbent sheet having the second absorbent material disposed between the second sheet and the second coating sheet.

In this case, by adding the second coating sheet, variations of the absorbent body can be increased. Moreover, the movement of the second absorbent material toward the outside of the second regions can be suppressed, so that the quality of the absorbent body is stabilized.

### EFFECTS OF THE INVENTION

According to the present invention, variations of the absorbent body can be increased.

### BRIEF DESCRIPTION OF THE DRAWINGS

[Figure 1] FIG. 1 is an explanatory view of an absorbent body manufacturing method (first embodiment).
[Figure 2] FIG. 2(a) is an explanatory view of first sheets and FIG. 2(b) is an explanatory view of a second sheet (first embodiment).
[Figure 3] FIG. 3(a) is a schematic plan view of a continuous body and an absorbent body and FIG. 3(b) is a schematic cross-sectional view of the continuous body and the absorbent body (first embodiment).
[Figure 4] FIG. 4 is a relevant part perspective view of the continuous body (first embodiment).
[Figure 5] FIG. 5 is an explanatory view of an absorbent body manufacturing method (second embodiment).
[Figure 6] FIG. 6 is an explanatory view of an absorbent body manufacturing method (third embodiment).
[Figure 7] FIG. 7(a) is a schematic plan view of a continuous body and an absorbent body and FIG. 7(b) is a schematic cross-sectional view of the continuous body and the absorbent body (third embodiment).
[Figure 8] FIG. 8 is an explanatory view of an absorbent body manufacturing method (fourth embodiment).
[Figure 9] FIG. 9(a) is a schematic plan view of a continuous body and an absorbent body and FIG. 9(b) is a schematic cross-sectional view of the continuous body and the absorbent body (fourth embodiment).
[Figure 10] FIG. 10 is the schematic layout diagram of the absorbent body manufacturing apparatus (first conventional example).
[Figure 11] FIG. 11 is the enlarged cross-sectional view of the relevant part of the absorbent body manufacturing apparatus (first conventional example).

### MODE FOR CARRYING OUT THE INVENTION

Hereinafter, embodiments of the present invention will be described with reference to the drawings.

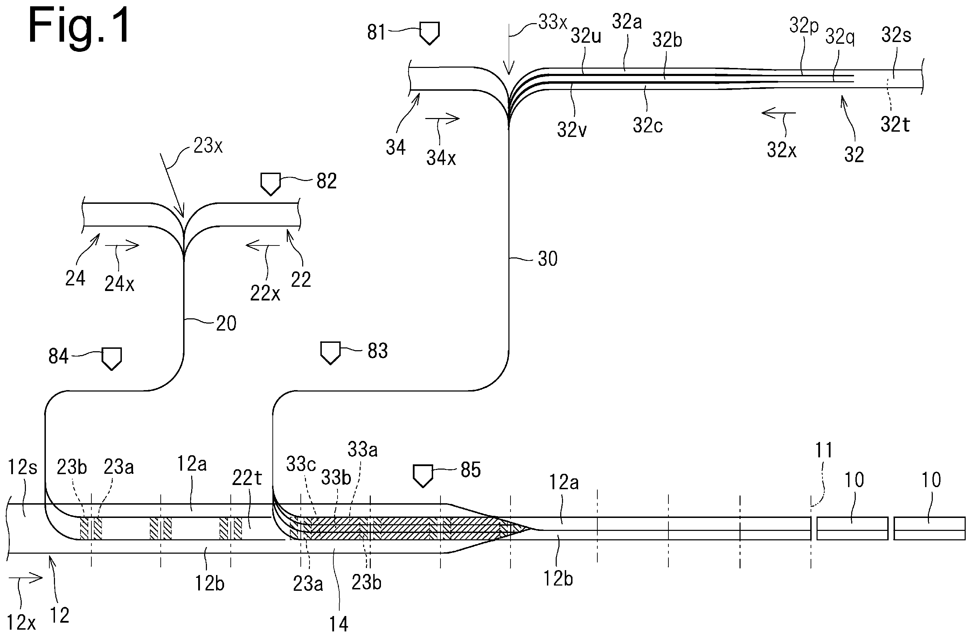

[First embodiment] An absorbent body manufacturing method of a first embodiment will be described with reference to FIGS. 1 to 4.

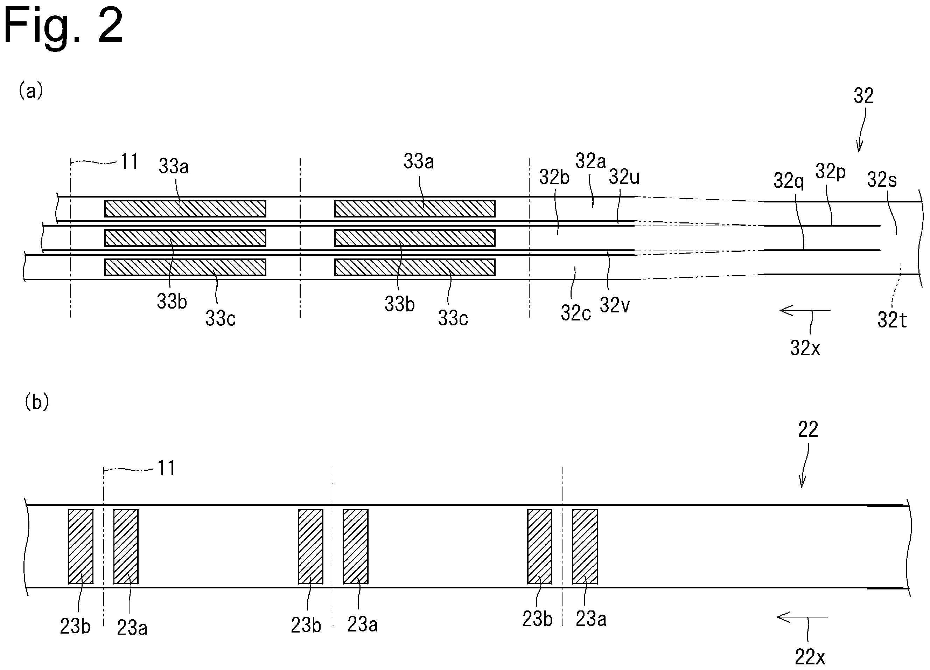

FIG. 1 is an explanatory view showing the outline of the method for manufacturing an absorbent body 10. As shown in FIG. 1, a first absorbent sheet 30 and a second absorbent sheet 20 are formed, a continuous body 14 is formed in which the first and second absorbent sheets 30 and 20 overlap each other, and then, the continuous body 14 is cut to form the absorbent body 10.
(1) The first absorbent sheet 30 is formed as follows: FIG. 2(a) is an explanatory view of first sheets 32a to 32c. As shown in FIGS. 1 and 2(a), a raw sheet 32 is conveyed in a direction 32x in which the raw sheet 32 continues, and a first coating sheet 34 is conveyed in a direction 34x in which the first coating sheet 34 continues. For example, the raw sheet 32 is air-laid nonwoven fabric, and the first coating sheet 34 is nonwoven fabric.

Then, slits 32p and 32q passing between principal surfaces 32s and 32t of the raw sheet 32 are formed to divide the raw sheet 32, thereby forming a plurality of first sheets 32a, 32b and 32c. Then, the spacings 32u and 32v between the adjoining first sheets 32a, 32b and 32c are increased, and the first sheets 32a to 32c are conveyed parallel to one another.

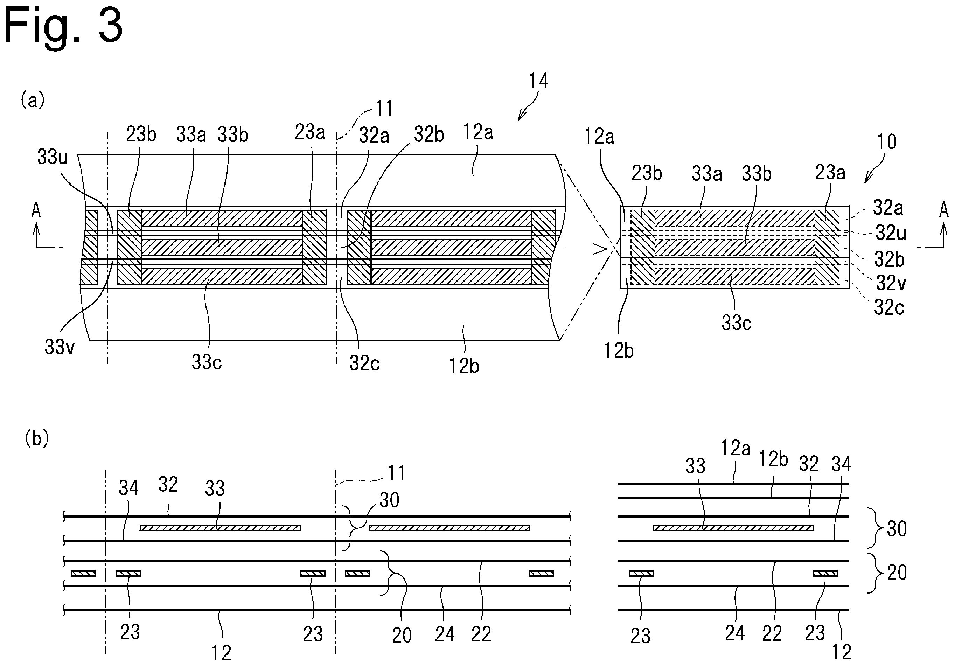

Then, as indicated by the arrow 33x in FIG. 1, a first absorbent material 33 (see FIG. 3(b)) is intermittently supplied to the first sheets 32a to 32c and/or the first coating sheet 34, and then, the first sheets 32a to 32c and the first coating sheet 34 are joined together to form the first absorbent sheet 30 having the first absorbent material 33 disposed. The first absorbent material 33 is an absorbent material that is granular or powdery in dry state, and is, for example, a powder and granular material of a superabsorbent polymer, abbreviated as "SAP".

The first absorbent sheet 30 can be formed, for example, by using the manufacturing apparatus of FIG. 10. In this case, the first sheets 32a to 32c are conveyed by using one of the conveyers C1 and C2, and the first coating sheet 34 is conveyed by using the other thereof. By using either one or both of the sprayers 111 and 112, the first absorbent material 33 is sprayed to the upper surface of the first sheets 32a to 32c and/or the first coating sheet 34 being conveyed along the inclined surfaces F1 and F2, and then, by using the laminating roller R, the first sheets 32a to 32c and the first coating sheet 34 are joined together. While the first absorbent sheet 30 can be formed without using the napping roller 130, the first sheets 32a to 32c may be napped by using the napping roller 130.

Hot melt or adhesive is previously applied to the first coating sheet 34 and/or the first sheets 32a and 32c by using an application device 81, and the first absorbent sheet 30 is formed where the first sheets 32a to 32c and the first coating sheet 34 are bonded together. The first absorbent sheet 30 may be formed where the bonding between the first sheets 32a and 32c and the first coating sheet 34 is omitted.

The first absorbent material 33 is supplied to the first sheets 32a to 32c and/or the first coating sheet 34 so as to form the first absorbent sheet 30 having the first absorbent material 33 disposed in hatched first regions 33a to 33c of the first sheets 32a to 32c shown in FIG. 2(a). The first regions 33a to 33c are aligned in the direction 32X in which the first sheets 32a to 32c continue, in a plurality of rows with a spacing therebetween. While the first absorbent material 33 is supplied at appropriate times at appropriate positions, it is preferable to supply it immediately before the first sheets 32a to 32c and the first coating sheet 34 are joined together because the disturbance of the supply pattern of the first absorbent material 33 can be suppressed.

The first regions 33a to 33c may be designed as appropriate; for example, they may be aligned in the direction 32x in which the first sheets 32a to 32c continue, or may continue without any spacing therebetween in the direction 32x in which the first sheets 32a to 32c continue. While the first regions 33a to 33c may intersect with a cutting position 11 where the continuous body 14 is to be cut to form the absorbent body 10, wear of the cutting blade can be suppressed if they are away from the cutting position 11 as shown in FIG. 2(a).

Instead of dividing a plurality of first sheets 32a to 32c from the raw sheet 32, a plurality of first sheets 32a to 32c prepared in advance may be used. The number of first sheets 32a to 32c may be one, two, or four or more.

Omitting the first coating sheet 34, the first absorbent sheet 30 may be formed where the first absorbent material 33 is disposed on the first sheets 32a to 32c. In this case, the spacings 32u and 32v between the first sheets 32a to 32c may be increased after the first absorbent material 33 is disposed on more than one of the first sheets 32a to 32c.

That is, the method for manufacturing the absorbent body 10 is provided with a first step of forming the first absorbent sheet 30 having the first absorbent material 33 disposed in the first regions 33a to 33c of at least one of the first sheets 32a to 32c.

Preferably, at the first step, the first absorbent material 33 is disposed on more than one of the first sheets 32a to 32c.

Preferably, at the first step, the first absorbent material 33 is disposed in the first regions 33a to 33c aligned in the direction 32x in which the first sheets 32a to 32c continue, in a plurality of rows with a spacing therebetween.

In a case where the first coating sheet 34 is not omitted, at the first step, the first coating sheet 34 is joined with the first sheets 32a to 32c to form the first absorbent sheet 30 having the first absorbent material 33 disposed between the first sheets 32a to 32c and the first coating sheet 34.

(2) The second absorbent sheet 20 is formed as follows: FIG. 2(b) is an explanatory view of a second sheet 22. As shown in FIGS. 1 and 2(b), the second sheet 22 is conveyed in a direction 22x in which the second sheet 22 continues, and a second coating sheet 24 is conveyed in a direction 24x in which the second coating sheet 24 continues. For example, the second sheet 22 and the second coating sheet 24 are nonwoven fabric.

Then, as indicated by the arrow 23x in FIG. 1, a second absorbent material 23 (see FIG. 3(b)) is intermittently supplied to the second sheet 22 and/or the second coating sheet 24. Then, the second sheet 22 and the second coating sheet 24 are joined together to form the second absorbent sheet 20. Like the first absorbent sheet 30, the second absorbent sheet 20 can be formed, for example, by using the manufacturing apparatus of FIG. 10. The second absorbent material 23 is an absorbent material that is granular or powdery in dry state, is, for example, a powder and granular material of SAP, and may be the same as or different from the first absorbent material 33.

Hot melt or adhesive is previously applied to the second sheet 22 and/or the second coating sheet 24 by using an application device 82, and the second absorbent sheet 20 is formed where the second sheet 22 and the second coating sheet 24 are bonded together. The second absorbent sheet 20 may be formed where the bonding between the second sheet 22 and the second coating sheet 24 is omitted.

The second absorbent material 23 is supplied to the second sheet 22 and/or the second coating sheet 24 so as to form the second absorbent sheet 20 having the second absorbent material 23 disposed in hatched second regions 23a and 23b of the second sheet 22 shown in FIG. 2(b). The second regions 23a and 23b are aligned in a row in the direction 22x in which the second sheet 22 continues, with a spacing therebetween. While the second absorbent material 23 is supplied at appropriate times at appropriate positions, it is preferable to supply it immediately before the second sheet 22 and the second coating sheet 24 are joined together because the disturbance of the supply pattern of the second absorbent material 23 can be suppressed.

The second absorbent material 23 may be designed as appropriate; for example, it may continue without any spacing, in the direction 22x in which the second sheet 22 continues. While the second regions 23a and 23b may intersect with the cutting position 11 where the continuous body 14 is to be cut to form the absorbent body 10, wear of the sheet cutting blade can be suppressed if they are away from the cutting position 11 as shown in FIG. 2(b).

Omitting the second coating sheet 24, the second absorbent sheet 20 may be formed where the second absorbent material 23 is disposed on the second sheet 22. An example of this structure will be described later as a fourth embodiment.

That is, the method for manufacturing the absorbent body 10 is provided with a second step of forming the second absorbent sheet 20 having the second absorbent material 23 disposed in the second regions 23a and 23b of the second sheet 22.

Preferably, at the second step, the second absorbent material 23 is disposed in the second regions 23a and 23b aligned with a spacing therebetween, in the direction 22x in which the second sheet 22 continues.

In a case where the second coating sheet 24 is not omitted, at the second step, the second coating sheet 24 is joined with the second sheet 22 to form the second absorbent sheet 20 having the second absorbent material 23 disposed between the second sheet 22 and the second coating sheet 24.

(3) The continuous body 14 and the absorbent body 10 are formed as follows: FIG. 3(a) is a schematic plan view showing the continuous body 14 in perspective and a schematic plan view of the absorbent body 10. FIG. 3(b) is a schematic cross-sectional view taken on the line A-A of FIG. 3(a). As shown in FIGS. 1 and 3, a carrier sheet 12 is conveyed in a direction 12x in which the carrier sheet 12 continues, and hot melt or adhesive is applied to the first and second absorbent sheets 30 and 20 by using application devices 83 and 84. Then, the second absorbent sheet 20 is placed on an intermediate region in the width direction of one principal surface 12s of the carrier sheet 12, and the second absorbent sheet 20 is pasted to the carrier sheet 12. Then, the first absorbent sheet 30 is placed on the second absorbent sheet 20, and the first absorbent sheet 30 is pasted to the second absorbent sheet 20. This forms the continuous body 14 including the first absorbent sheet 30 and the second absorbent sheet 20.

At this time, the first and second absorbent sheets 30 and 20 are placed one on another so that the first regions 33a to 33c in which the first absorbent material 33 is disposed on the first absorbent sheet 30 and the second regions 23a and 23b in which the second absorbent material 23 is disposed on the second absorbent sheet 20 are adjacent to each other in plan view, and the first regions 33a to 33c and the second regions 23a and 23b adjacent to each other are arranged between the adjoining cutting positions 11.

Then, hot melt or adhesive is applied to the carrier sheet 12 and/or the first absorbent sheet 30 by using an application device 85, and then, parts 12a and 12b of the carrier sheet 12 protruding on both sides of the first and second adhesive sheets 30 and 20 are folded and placed on the first absorbent sheet 30. Thereby, the continuous body 14 has the first and second absorbent sheets 30 and 20 wrapped by the carrier sheet 12.

Then, the continuous body 14 is cut at the cutting positions 11 to form the absorbent body 10. Thereby, the absorbent body 10 can be formed in which the first and second absorbent materials 23 and 33 are disposed.

Some or all of the application apparatuses 81 to 85 may be omitted. Appropriate parts may be bonded at appropriate times by ultrasonic waves, heat sealing or the like instead of adhesive bonding. The first and/or second absorbent sheets 30 and 20 and the continuous body 14 may be formed with adhesive bonding or adhesive-free bonding omitted.

The order in which the carrier sheet 12 and the first and second absorbent sheets 30 and 20 are placed one on another is arbitrary. For example, the order may be as follows: The second absorbent sheet 20 is placed on the carrier sheet 12, and then, the first absorbent sheet 30 is placed on the second absorbent sheet 20. After the first and second absorbent sheets 30 and 20 are placed one on another, the first and second absorbent sheets 30 and 20 are placed on the carrier sheet 12. Further, the continuous body 14 may be formed in which the carrier sheet 12 is omitted and the first and second absorbent sheets 30 and 20 overlap each other.

That is, the method for manufacturing the absorbent body 10 is provided with: a third step of forming the continuous body 14 including the first absorbent sheet 30 and the second absorbent sheet 20 and in which the first regions 33a to 33c and the second regions 23a and 23b are adjacent to each other in plan view; and a fourth step of forming the absorbent body 10 including the first absorbent material 33 and the second absorbent material 23, by cutting the continuous body 14 so that the first regions 33a to 33c and the second regions 23a and 23b adjacent to each other are included.

Specifically, at the third step, the continuous body 14 is formed in which the first absorbent sheet 30 and the second absorbent sheet 20 overlap each other such that the first regions 33a to 33c and the second regions 23a and 23b are adjacent to each other in plan view. In this case, the overlapping first and second absorbent sheets 30 and 20 are simultaneously cut at the fourth step to form the absorbent body.

Preferably, at the third step, the continuous body 14 is formed in which more than one of the first sheets 32a to 32c is arranged with a spacing therebetween.

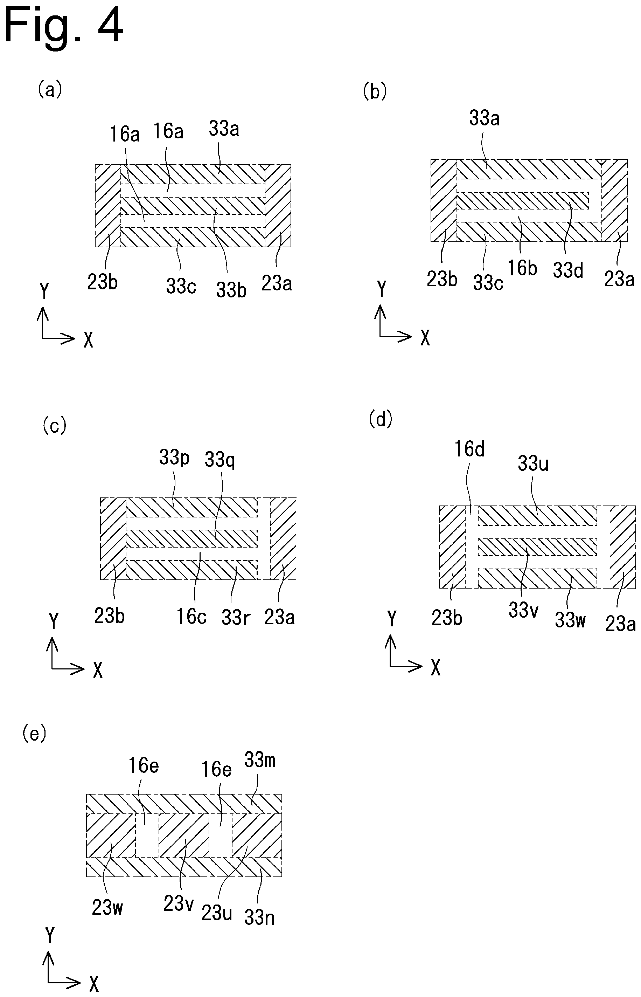

(4) Next, the disposition pattern of the first and second regions adjacent to each other in plan view will be described with reference to FIG. 4. FIG. 4 is a relevant part perspective view of the continuous body 14 in which the first and second absorbent sheets 30 and 20 overlap each other, and show the first and second regions arranged between the adjoining cutting positions 11. The first and second absorbent sheets 30 and 20 continue in a first direction X.

FIG. 4(a) is the same disposition pattern as that of FIGS. 1 to 3. As shown in FIG. 4(a), the three first regions 33a to 33c extending in the first direction X are arranged between the two second regions 23a and 23b arranged with a spacing therebetween in the first direction X, and the first regions 33a to 33c are in contact with the two second regions 23a and 23b. The three first regions 33a to 33c are arranged with a spacing therebetween in a second direction Y orthogonal to the first direction X. This forms two channels 16a surrounded by the first and second regions 33a to 33c, and 23a and 23b. The first and second absorbent materials 33 and 22 are not disposed in the two channels 16a.

In FIG. 4(b), the three first regions 33a, 33d and 33c extending in the first direction X are arranged between the two second regions 23a and 23b arranged with a spacing therebetween in the first direction X. Of the three first regions 33a, 33d and 33c, the first regions 33a and 33c on both side in the second direction Y are in contact with the two second regions 23a and 23b, and the central first region 33d is in contact with only one second region 23b and adjoins the other second region 23a with a spacing therebetween. This forms one U-shaped channel 16b.

In FIG. 4(c), three first regions 33p to 33r extending in the first direction X are arranged between the two second regions 23a and 23b arranged with a spacing therebetween in the first direction X, and the three first regions 33p to 33r each adjoin the one second region 23a with a spacing therebetween and are in contact with the other second region 23b. This forms a pi-shaped channel 16c that is open at two positions on both sides in the second direction Y.

In FIG. 4(d), three first regions 33u to 33w extending in the first direction X are arranged between the two second regions 23a and 23b arranged with a spacing therebetween in the first direction X, and the three first regions 33u to 33w each adjoin the two second regions 23a and 23b with a spacing from the two second regions 23a and 23b. This forms one substantially II-shaped or substantially H-shaped channel 16d that is open at four positions on both sides in the second direction Y.

In FIG. 4(e), two first regions 33m and 33n extending in the first direction X are arranged with a spacing therebetween in the second direction Y, three second regions 23u to 23w are arranged with a spacing therebetween in the first direction X between the two first regions 33m and 33n, and the three second regions 23u to 23w each adjoin the two first regions 33m and 33n. This forms two channels 16e surrounded by the first and second regions 33m and 33n, and 23u to 23w, and the two channels 16e are aligned in the first direction X.

The disposition pattern is possible in which the first regions and the second regions shown in FIG. 4 are interchanged.

By forming the absorbent body by the above-described method, it is possible to change the characteristics of the absorbent body according to the pattern of the first and second regions and the kind and amount of the first and second absorbent materials disposed in the first and second regions. Consequently, variations of the absorbent body can be increased.

When the absorbent body 10 includes a plurality of first sheets 32a and 32c, a channel can be formed along the spacings 32u and 32v between the adjoining first sheets 32a to 32c. Moreover, because the plural first sheets 32a to 32c are not restrained by one another, an absorbent body 10 can be formed that easily follows the shape of the wearing part.

By adding the first coating sheet 34, the movement of the first absorbent material 33 toward the outside of the first regions 33a and 33b is suppressed by the first coating sheet 34, so that the quality of the absorbent body 10 is stabilized. By adding the second coating sheet 24, the movement of the second absorbent material 23 toward the outside of the second regions 23a and 23b is suppressed by the second coating sheet 24, so that the quality of the absorbent body 10 is stabilized. By selecting various characteristics such as liquid permeability, liquid diffusibility and liquid retainability of the first and/or second coating sheets 33 and 23, variations of the absorbent body 10 can be increased.

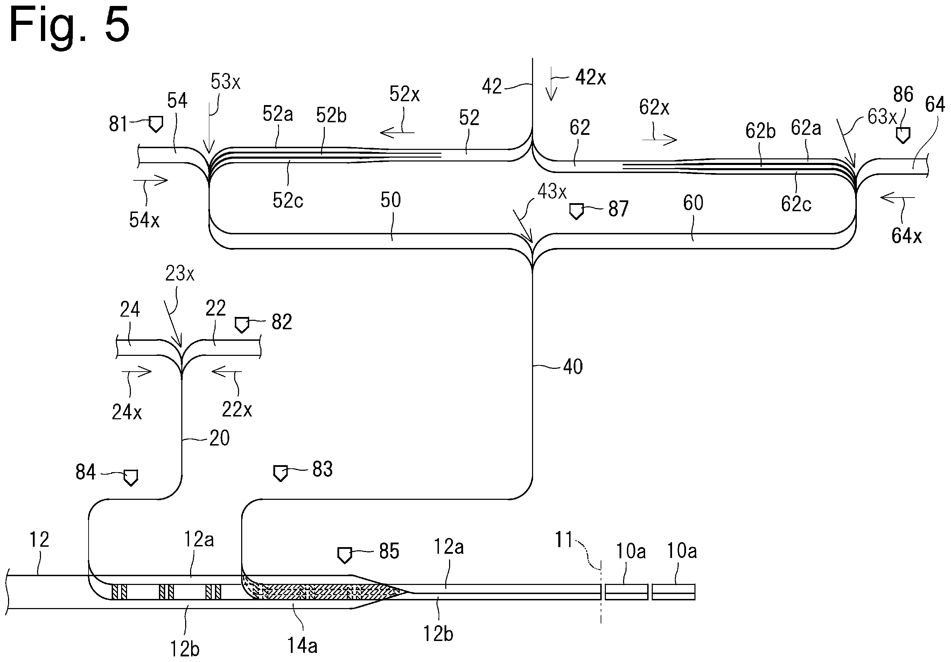

[Second embodiment] A method for manufacturing an absorbent body 10a of a second embodiment with an increased number of sheets will be described with reference to FIG. 5. FIG. 5 is an explanatory view showing the outline of the method for manufacturing the absorbent body 10a. Hereinafter, the same parts as those of the first embodiment are denoted by the same reference designations and differences from the first embodiment will mainly be described.

As shown in FIG. 5, the absorbent body manufacturing method of the second embodiment is substantially similar to that of the first embodiment.

A raw sheet 42 is conveyed in a direction 42x in which the raw sheet 42 continues, and the raw sheet 42 is divided into first and third divisional sheets 52 and 62.

As in the first embodiment, the first divisional sheet 52 is conveyed in a direction 52x in which the first divisional sheet 52 continues, and a first coating sheet 54 is conveyed in a direction 54x in which the first coating sheet 54 continues. The first divisional sheet 52 is divided to form a plurality of first sheets 52a to 52c. Then, as indicated by the arrow 53x, the first absorbent material is supplied to the first sheets 52a to 52c and/or the first coating sheet 54. Then, the first sheets 52a to 52c and the first coating sheet 54 are joined together to form a first absorbent sheet 50 where the first absorbent material is sandwiched between the first sheets 52a to 52c and the first coating sheet 54 and the first absorbent material is disposed in first regions of the first sheets 52a to 52c.

Likewise, the third divisional sheet 62 is conveyed in a direction 62x in which the third divisional sheet 62 continues, and a third coating sheet 64 is conveyed in a direction 64x in which the third coating sheet 64 continues. Then, the third divisional sheet 62 is divided to form a plurality of third sheets 62a to 62c. Then, as indicated by the arrow 63x, a third absorbent material is supplied to the third sheets 62a to 62c and/or the third coating sheet 64. Then, the third sheets 62a to 62c and the third coating sheet 64 are joined together to form a third absorbent sheet 60 where the third absorbent material 63 is sandwiched between the third sheets 62a to 62c and the third coating sheet 64 and the third absorbent material is disposed in third regions of the third sheet 60.

Then, as indicated by the arrow 43x, a fourth absorbent material is supplied to the first absorbent sheet 50 and/or the third absorbent sheet 60. Then, the first and third absorbent sheets 50 and 60 are joined together to form a fourth absorbent sheet 40 where the fourth absorbent material is sandwiched between the first absorbent sheet 50 and the third absorbent sheet 60 and the fourth absorbent material is disposed in fourth regions.

The second sheet 22 is conveyed in the direction 22x in which the second sheet 22 continues, the second coating sheet 24 is conveyed in the direction 24x in which the second coating sheet 24 continues, the second absorbent material is supplied to the second sheet 22 and/or the second coating sheet 24 as indicated by the arrow 23x, and the second sheet 22 and the second coating sheet 24 are joined together to form the second absorbent sheet 20 where the second absorbent material is sandwiched between the second sheet 22 and the second coating sheet 24 and the second absorbent material is disposed in the second regions of the second sheet 22.

Then, the second absorbent sheet 20 is placed on the carrier sheet 12, and then, the fourth absorbent sheet 40 is placed on the second absorbent sheet 20. This forms a continuous body 14a including the second absorbent sheet 20 and the fourth absorbent sheet 40. Since the fourth absorbent sheet 40 includes the first absorbent sheet 50, the continuous body 14a includes the first absorbent sheet 50 and the second absorbent sheet 20. At this time, the continuous body 14a is formed in which at least two of the first to fourth regions are adjacent to each other in plan view. For example, the fourth absorbent sheet 40 and the continuous body 14a are formed so that two of the first to fourth regions are adjacent to each other and the other two regions overlap either one or both of the adjoining two regions in plan view. The continuous body 14a may be formed so that none of the first to fourth regions overlaps the other regions in plan view. Then, both side parts 12a and 12b of the carrier sheet 12 in the width direction are folded. Thereby, the second absorbent sheet 20 and the fourth absorbent sheet 40 are wrapped by the carrier sheet 12.

Then, the continuous body 14a is cut to form the absorbent body 10a. At this time, the absorbent body 10a including at least two of the first to fourth absorbent materials is formed by cutting the continuous body 14a at the cutting positions 11 so that at least two adjoining regions are included between the adjoining cutting positions 11.

As in the first embodiment, the sheets are bonded as appropriate by applying hot melt or adhesive by using the application devices 81 to 87. Appropriate parts may be bonded at appropriate times by ultrasonic waves, heat sealing or the like instead of adhesive bonding. The first to fourth absorbent sheets 50, 20, 60 and 40 and the continuous body 14a may be formed with adhesive bonding or adhesive-free bonding omitted.

With the absorbent body 10a, compared with the first embodiment, the kind and amount of the absorbent materials and the number of sheets can be increased. Consequently, variations of the absorbent body can be increased.

In the second embodiment, as in the first embodiment, one or more of the first to third coating sheets 54, 24 and 64 may be omitted. The number of first and/or third sheets 52a to 52c and 62a to 63c may be one, two, or four or more. The continuous body 14 may be formed in which the second and fourth absorbent sheets 20 and 40 overlap each other with the carrier sheet 12 omitted.

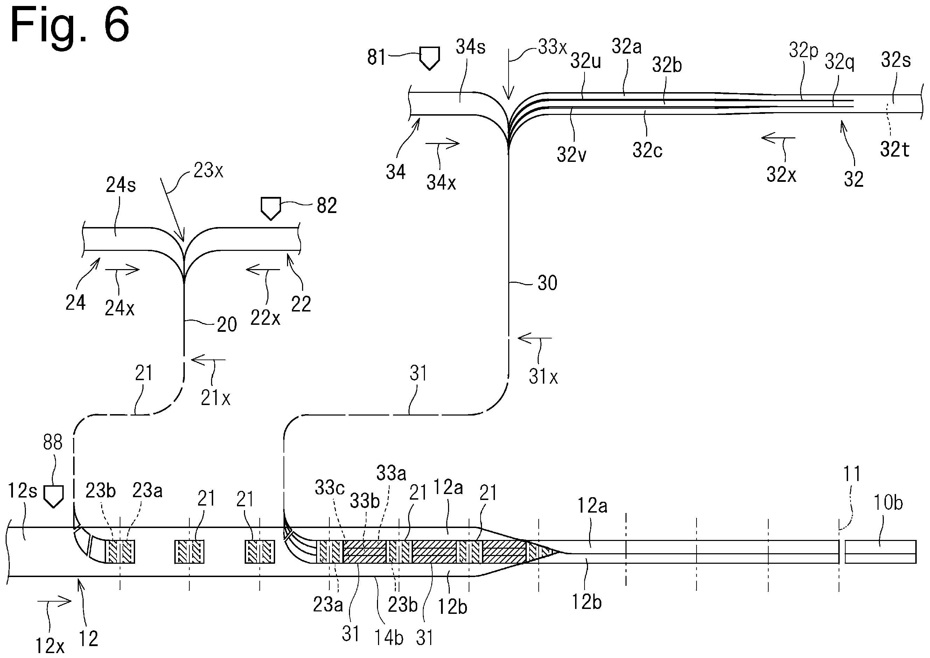

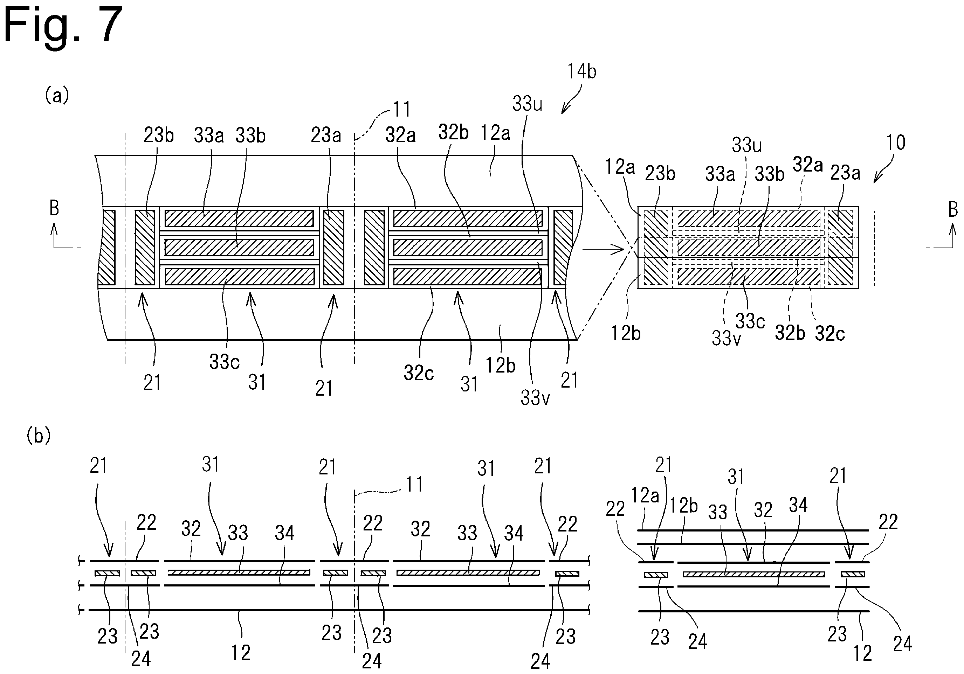

[Third embodiment] A method for manufacturing an absorbent body 10b of a third embodiment in which the first and second absorbent sheets 30 and 20 do not overlap each other will be described with reference to FIGS. 6 and 7. FIG. 6 is an explanatory view of the method for manufacturing the absorbent body 10b of the third embodiment. FIG. 7(a) is a schematic plan view of the continuous body 14b and the absorbent body 10b. FIG. 7(b) is a schematic cross-sectional view taken on the line B-B of FIG. 7(a).

As shown in FIG. 6, as in the first embodiment, the following are formed: the first absorbent sheet 30 where the first absorbent material 33 is sandwiched between the first sheets 32a to 32c and the first coating sheet 34 and the first absorbent material 33 is disposed in the first regions 33a to 33c of the first sheets 32a to 32c; and the second absorbent sheet 20 where the second absorbent material 23 is sandwiched between the second sheet 22 and the second coating sheet 24 and the second absorbent material 23 is disposed in the second regions 23a and 23b of the second sheet 22.

Then, unlike the first embodiment, as indicated by the arrow 31x, the first absorbent sheet 30 is cut to form first pieces 31 including the first regions 33a to 33c and the first absorbent material 33. Moreover, as indicated by the arrow 21x, the second absorbent sheet 20 is cut to form second pieces 21 including the second regions 23a and 23b and the second absorbent material 23.

Then, as shown in FIGS. 6 and 7, hot melt or adhesive is applied to one principal surface 12s of the carrier sheet 12 by using an application device 88, and then, the second pieces 21 are pasted to a central region of the carrier sheet 12 with a predetermined spacing. Then, the first pieces 31 are pasted to the regions between the second pieces 21 of the carrier sheet 12. This forms the continuous body 14b including the first pieces 31 and the second pieces 21. At this time, the first pieces 31 and the second pieces 21 are aligned such that the first regions 31 and the second regions 21 are adjacent to each other in plan view.

Then, parts 12a and 12b of the carrier sheet 12 on both sides in the width direction are folded and placed on the first absorbent sheet 30, so that the first pieces 31 and the second pieces 21 are wrapped by the carrier sheet 12.

Then, the continuous body 14b is cut at the cutting positions 11 to form the absorbent body 10b. Thereby, the absorbent body 10b can be formed in which the first pieces 31 of the first absorbent sheet 30 and the second pieces 21 of the second absorbent sheet 20 are arranged.

As described above, as in the first embodiment, the method for manufacturing the absorbent body 10b of the third embodiment is provided with: a first step of forming the first absorbent sheet 30 having the first absorbent material 33 disposed in the first regions 33a to 33c of at least one of the first sheets 32a to 32c; a second step of forming the second absorbent sheet 20 having the second absorbent material 23 disposed in the second regions 23a and 23b of the second sheet 22; a third step of forming the continuous body 14b including the first absorbent sheet 30 and the second absorbent sheet 20 and in which the first regions 33a to 33c and the second regions 23a and 23b are adjacent to each other in plan view; and a fourth step of forming the absorbent body 10b including the first absorbent material 33 and the second absorbent material 23 by cutting the continuous body 14b so that the first regions 33a to 33c and the second regions 23a and 23b adjacent to each other are included.

Specifically, unlike the first embodiment, the method of the third embodiment is further provided with: a fifth step of forming the first pieces 31 including the first regions 33a to 33c, by cutting the first absorbent sheet 30 after the first step and before the third step; and a sixth step of forming the second pieces 21 including the second regions 23a and 23b, by cutting the second absorbent sheet 20 after the second step and before the third step. At the third step, the continuous body 14b is formed in which the first pieces 31 and the second pieces 21 are aligned such that the first regions 33a to 33c and the second regions 23a and 23b are adjacent to each other in plan view.

As in the first embodiment, preferably, at the first step, the first absorbent material 33 is disposed on more than one of the first sheets 32a to 32c. At the third step, the continuous body 14 is formed in which more than one the first sheets 32a to 32c is arranged with a spacing therebetween.

Moreover, as in the first embodiment, preferably, at the first step, the first absorbent material 33 is disposed in the first regions 33a to 33c aligned in the direction 32x in which the first sheets 32a to 32c continue, in a plurality of rows with a spacing therebetween. At the second step, the second absorbent material 23 is disposed in the second regions 22a and 22b aligned in the direction 22x in which the second sheet 22 continues, with a spacing therebetween.

Moreover, as in the first embodiment, preferably, at the first step, the first coating sheet 34 is joined with the first sheets 32a to 32c to form the first absorbent sheet 30 having the first absorbent material 33 disposed between the first sheets 32a to 32c and the first coating sheet 34.

Moreover, as in the first embodiment, preferably, at the second step, the second coating sheet 24 is joined with the second sheet 22 to form the second absorbent sheet 20 having the second absorbent material 23 disposed between the second sheet 22 and the second coating sheet 24.

According to the absorbent body manufacturing method of the third embodiment, since the continuous body 14b is formed in which the first and second absorbent sheets 30 and 20 do not overlap each other, compared with the first embodiment, the thickness of the absorbent body 10b can be reduced, and the sheet materials used for the absorbent body 10b can be reduced.

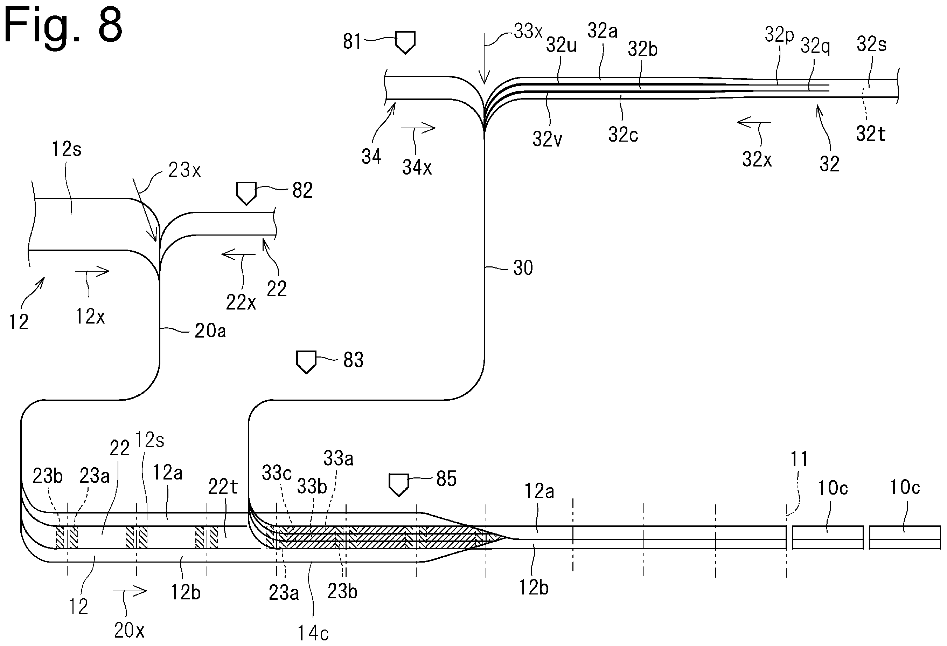

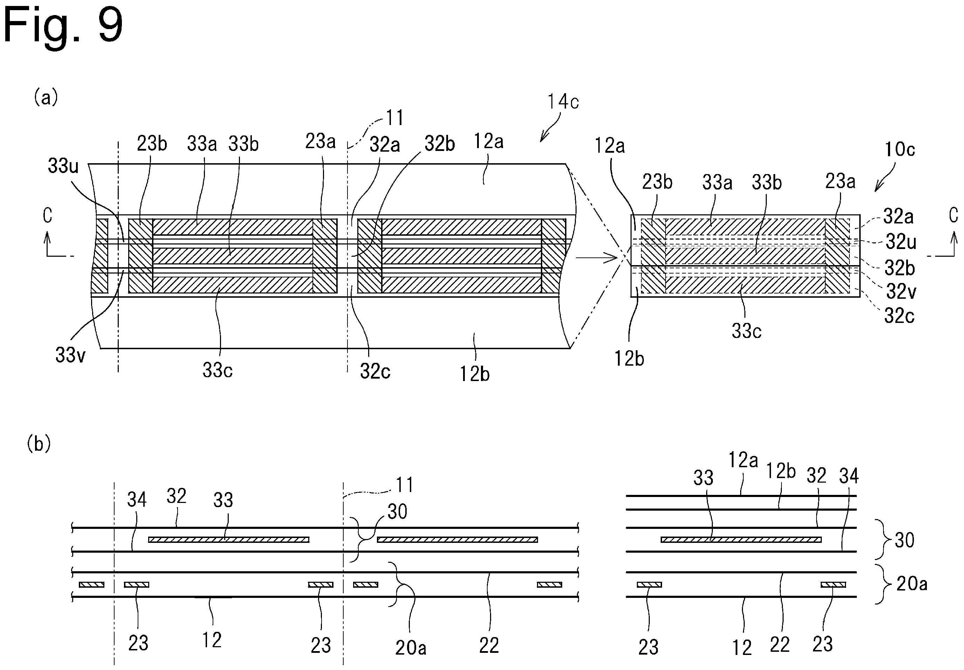

[Fourth embodiment] An absorbent body manufacturing method of a fourth embodiment will be described with reference to FIGS. 8 and 9. In the fourth embodiment, an absorbent body 10c is manufactured with the second coating sheet 24 of the first embodiment omitted. FIG. 8 is an explanatory view showing the outline of the method for manufacturing the absorbent body 10c. FIG. 9(a) is a schematic plan view of a continuous body 14c and the absorbent body 10c, and FIG. 9(b) is a schematic cross-sectional view taken on the line C-C of FIG. 9(a).

As shown in FIGS. 8 and 9, as in the first embodiment, the first absorbent sheet 30 is formed where the first absorbent material 33 is sandwiched between the first sheets 32a to 32c and the first coating sheet 34 and the first absorbent material 33 is disposed in the first regions 33a to 33c of the first sheets 32a to 32c.

The second absorbent sheet 20a is formed in a similar manner to that of the first embodiment by using the carrier sheet 12 instead of the second coating sheet 24 of the first embodiment. That is, the second sheet 22 is conveyed in the direction 22x in which the second sheet 22 continues, and the carrier sheet 12 wider than the second sheet 22 is conveyed in the direction 12x in which the carrier sheet 12 continues. Then, as indicated by the arrow 23x in FIG. 8, the second absorbent material 23 is intermittently supplied to the second sheet 22 and/or the carrier sheet 12. Then, the second sheet 22 is placed on a central region in the width direction of one principal surface 12s of the carrier sheet 12 to form the second absorbent sheet 20a. This forms the second absorbent sheet 20a where the second absorbent material 23 is sandwiched between the second sheet 22 and the carrier sheet 12 and the second absorbent material 23 is disposed in the second regions 23a and 23b of the second sheet 22.

Then, the second absorbent sheet 20a is conveyed in a direction 20x in which the second absorbent sheet 20a continues. Then, the first absorbent sheet 30 having hot melt or adhesive applied thereto by using the application device 83 is pasted to the second absorbent sheet 20a so as to overlap the second sheet 22 of the second absorbent sheet 20a. This forms the continuous body 14c including the first absorbent sheet 30 and the second absorbent sheet 20a.

At this time, the first and second absorbent sheets 30 and 20a are placed one on another so that the first regions 33a to 33c having the first absorbent material 33 disposed on the first absorbent sheet 30 and the second regions 23a and 23b having the second absorbent material 23 disposed on the second absorbent sheet 20a are adjacent to each other in plan view, to make the first regions 33a to 33c and the second regions 23a and 23c arranged between the adjoining cutting positions 11.

Then, hot melt or adhesive is applied to the second absorbent sheet 20a and/or the first absorbent sheet 30 by using the application device 85, and then, the parts 12a and 12b of the carrier sheet 12 protruding on both sides of the first absorbent sheet 30 are folded and placed on the first absorbent sheet 30. Thereby, the continuous body 14c has the first and second absorbent sheets 30 and 20a wrapped by the carrier sheet 12.

Then, the continuous body 14c is cut at the cutting positions 11 to form the absorbent body 10c. Thereby, the absorbent body 10c can be formed in which the first and second absorbent materials 23 and 33 are disposed.

Some or all of the application apparatuses 81 to 83 and 85 may be omitted. Appropriate parts may be bonded at appropriate times by ultrasonic waves, heat sealing or the like instead of adhesive bonding. The first and/or second absorbent sheets 30 and 20a and the continuous body 14c may be formed with adhesive bonding or adhesive-free bonding omitted.

In the fourth embodiment, variations of the absorbent body 10c can be increased by omitting the second coating sheet 24. Moreover, the materials used for manufacturing the absorbent body 10c can be reduced.

[Summary] The above-described absorbent body manufacturing methods are capable of increasing variations of the absorbent body.

The present invention is not limited to the above-described embodiments but may be carried out with various modifications being added.

For example, the first to fourth sheets may be other than nonwoven fabric. Moreover, the absorbent body may be formed by forming the continuous body by cutting either one of the first and second absorbent sheets into pieces without cutting the other one thereof into pieces and then, cutting the continuous body.

### DESCRIPTION OF REFERENCE NUMERALS

10, 10a, 10b, 10c Absorbent body
14, 14a, 14b, 14c Continuous body
20, 20a Second absorbent sheet
21 Second piece
22 Second sheet
23 Second absorbent material
23a, 23b, 23u-23w Second region
24 Second coating sheet
30 First absorbent sheet
31 First piece
32a-32c First sheet
33 First absorbent material
33a-33d, 33m, 33n, 33p-33r, 33u-33w First region
34 First coating sheet

## Claims

1. An absorbent body manufacturing method comprising:
a first step of forming a first absorbent sheet (30; 50) having a first absorbent material (33) disposed in first regions (33a-33d, 33p-33r, 33u-33w; 33m, 33n) of a first sheet (32a-32c; 52a-52c);
a second step of forming a second absorbent sheet (20, 20a) having a second absorbent material (23) disposed in second regions (23a, 23b; 23u-23w) of a second sheet (22);
a third step of forming a continuous body (14, 14a, 14b, 14c) including the first absorbent sheet (30, 50) and the second absorbent sheet (20, 20a) and in which the first regions (33a-33d, 33p-33r, 33u-33w; 33m, 33n) and the second regions (23a, 23b; 23u-23w) are adjacent to each other in plan view; and
a fourth step of forming an absorbent body (10, 10a, 10b, 10c) including the first absorbent material (33) and the second absorbent material (23), by cutting the continuous body (14, 14a, 14b, 14c) so that the first regions (33a-33d, 33p-33r, 33u-33w; 33m, 33n) and the second regions (23a, 23b; 23u-23w) adjacent to each other are included,
**characterized in that**
at the first step, the first absorbent material (33) is disposed in the first regions (33a-33d, 33p-33r, 33u-33w; 33m, 33n) aligned in a direction in which the first sheet (32a-32c) continues, in a plurality of rows with a spacing therebetween, and
at the second step, the second absorbent material (23) is disposed in the second regions (23a, 23b; 23u-23w) aligned in a direction in which the second sheet (22) continues, with a spacing therebetween.

2. The absorbent body manufacturing method according to claim 1, wherein at the third step, the continuous body (14, 14a, 14b, 14c) is formed in which the first absorbent sheet (30, 50) and the second absorbent sheet (20, 20a) overlap each other such that the first regions (33a-33d, 33p-33r, 33u-33w; 33m, 33n) and the second regions (23a, 23b; 23u-23w) are adjacent to each other in plan view.

3. The absorbent body manufacturing method according to claim 1, further comprising:
a fifth step of forming first pieces (31) including the first regions (33a-33d, 33p-33r, 33u-33w; 33m, 33n), by cutting the first absorbent sheet (30) after the first step and before the third step; and
a sixth step of forming second pieces (21) including the second region (23a, 23b; 23u-23w), by cutting the second absorbent sheet (20) after the second step and before the third step,
wherein at the third step, the continuous body (14b) is formed in which the first pieces (31) and the second pieces (21) are aligned such that the first regions (33a-33c) and the second regions (23a, 23b) are adjacent to each other in plan view.

4. The absorbent body manufacturing method according to any one of claims 1 to 3, wherein at the first step, the first absorbent material (33) is disposed on more than one of the first sheets (32a-32c; 52a-52c), and
at the third step, the continuous body(14, 14a, 14b, 14c) is formed in which more than one of the first sheets (32a-32c; 52a-52c) is arranged with a spacing therebetween.

5. The absorbent body manufacturing method according to any one of claims 1 to 4, wherein at the first step, a first coating sheet (34, 54) is joined with the first sheet (32a-32c, 52a-52c) to form the first absorbent sheet (30, 50) having the first absorbent material (33) disposed between the first sheet (32a-32c, 52a-52c) and the first coating sheet (34, 54).

6. The absorbent body manufacturing method according to any one of claims 1 to 5, wherein at the second step, a second coating sheet (24) is joined with the second sheet (22) to form the second absorbent sheet (20) having the second absorbent material (23) disposed between the second sheet (22) and the second coating sheet (24).

## Patentansprüche

1. Ein Verfahren zur Herstellung von absorbierenden Körpern, das umfasst:
ein erster Schritt zur Bildung eines ersten absorbierenden Blatts (30; 50), wobei ein erstes saugfähiges Material (33) in den ersten Bereichen (33A-33D, 33P-33R, 33U-33W; 33M, 33N) eines ersten Blattes (32A-32C; 52A-52C) angebracht wird;
ein zweiter Schritt zur Bildung eines zweiten Absorptionsblatts (20, 20a), wobei ein zweites Absorptionsmaterial (23) in zweiten Bereichen (23A, 23B; 23U-23W) eines zweiten Blattes (22) angeordnet wird;
Ein dritter Schritt zur Bildung eines durchgehenden Körpers (14, 14A, 14B, 14C), einschließlich der ersten Absorptionsschicht (30, 50) und der zweiten Absorptionsschicht (20, 20a), wobei die ersten Regionen (33A-33D, 33P-33R, 33U-33W; 33m, 33n) und die zweiten Regionen (23A, 23B; 23U-23W) im Grundriss nebeneinander liegen; und
Ein vierter Schritt: die Bildung eines absorbierenden Körpers (10, 10a, 10b, 10c), der das erste absorbierende Material (33) und das zweite absorbierende Material (23) umfasst, indem der kontinuierliche Körper (14, 14a, 14b, 14c) so geschnitten wird, dass die ersten Regionen (33a-33d, 33p-33r, 33u-33w; 33m, 33n) und die zweiten Regionen (23a, 23b; 23u-23w) nebeneinander eingeschlossen sind,
Charakterisiert durch
Im ersten Schritt wird das erste absorbierende Material (33) in den ersten Bereichen (33A-33D, 33P-33R, 33U-33W; 33M, 33N) in einer Richtung angelegt, in der das erste Blatt (32A-32C) fortgesetzt wird, in mehreren Reihen mit Abstand dazwischen, und
Im zweiten Schritt wird das zweite absorbierende Material (23) in den zweiten Bereichen (23a, 23b; 23u-23w) in eine Richtung gelegt, in der das zweite Blatt (22) mit einem Abstand dazwischen weiterläuft.

2. Das Verfahren zur Herstellung von Absorptionskörpern gemäß Anspruch 1, bei dem im dritten Schritt der kontinuierliche Körper (14, 14a, 14b, 14c) gebildet wird, bei dem das erste Absorptionsblatt (30, 50) und das zweite Absorptionsblatt (20, 20a) sich so überlappen, dass die ersten Regionen (33a-33d, 33p-33r, 33u-33w; 33m, 33n) und die zweiten Regionen (23a, 23b; 23U-23W) im Grundriss nebeneinanderliegen.

3. Das Verfahren zur Herstellung von absorbierenden Körpern gemäß Anspruch 1, das weiter:
Ein fünfter Schritt besteht darin, die ersten Teile (31) einschließlich der ersten Regionen (33A-33D, 33P-33R, 33U-33W; 33M, 33N) zu bilden, indem das erste Saugblatt (30) nach dem ersten Schritt und vor dem dritten Schritt zugeschnitten wird; und
Ein sechster Schritt besteht darin, zweite Stücke (21) einschließlich des zweiten Bereichs (23a, 23B; 23U-23W) zu bilden, indem das zweite Absorptionsblatt (20) nach dem zweiten Schritt und vor dem dritten Schritt geschnitten wird,
wobei im dritten Schritt der kontinuierliche Körper (14b) gebildet wird, in dem die ersten Teile (31) und die zweiten Teile (21) so ausgerichtet sind, dass die ersten Regionen (33a-33c) und die zweiten Regionen (23a, 23b) in der Grundrissansicht nebeneinander liegen.

4. Das Verfahren zur Herstellung von absorbierenden Körpern gemäß einem der Ansprüche 1 bis 3, bei dem im ersten Schritt das erste saugfähige Material (33) auf mehr als einem der ersten Blätter (32a-32c; 52a-52c) entsorgt wird, und
Im dritten Schritt wird der durchgehende Körper (14, 14A, 14B, 14C) gebildet, in dem mehr als eine der ersten Platten (32A-32C; 52A-52C) mit einem Abstand dazwischen angeordnet ist.

5. Das Verfahren zur Herstellung von Absorptionskörpern gemäß einem der Ansprüche 1 bis 4, wobei im ersten Schritt eine erste Beschichtungsschicht (34, 54) mit der ersten Schicht (32A-32C, 52A-52C) verbunden wird, um die erste Saugfolie (30, 50) zu bilden, wobei das erste Saugmaterial (33) zwischen der ersten Schicht (32A-32C, 52A-52C) und der ersten Beschichtungsschicht (34, 54).

6. Das Saugkörperherstellungsverfahren gemäß einem der Ansprüche 1 bis 5, wobei im zweiten Schritt eine zweite Beschichtungsschicht (24) mit der zweiten Schicht (22) verbunden wird, um die zweite Saugschicht (20) zu bilden, wobei das zweite Saugmaterial (23) zwischen der zweiten Schicht (22) und der zweiten Beschichtungsschicht (24) angeordnet wird.

## Revendications

1. Une méthode de fabrication de corps absorbant comprenant :
une première étape consiste à former une première feuille absorbante (30 ; 50) avec un premier matériau absorbant (33) disposé en premières régions (33A-33D, 33P-33R, 33U-33W ; 33M, 33N) d'une première feuille (32A-32C ; 52A-52C) ;
une seconde étape consistant à former une seconde feuille absorbante (20, 20a) avec un second matériau absorbant (23) disposé en secondes régions (23a, 23b ; 23U-23W) d'une seconde feuille (22) ;
une troisième étape consiste à former un corps continu (14, 14a, 14b, 14c) incluant la première feuille absorbante (30, 50) et la seconde feuille absorbante (20, 20a), et où les premières régions (33A-33D, 33P-33R, 33U-33W ; 33M, 33N) et les secondes régions (23A, 23B ; 23U-23W) sont adjacentes en vue en plan ; et
Une quatrième étape consiste à former un corps absorbant (10, 10A, 10B, 10C) incluant le premier matériau absorbant (33) et le second matériau absorbant (23), en découpant le corps continu (14, 14A, 14B, 14C) afin que les premières régions (33A-33D, 33P-33R, 33U-33W ; 33M, 33N) et les secondes régions (23A, 23B ; 23U-23W) adjacentes soient incluses,
caractérisée en ce
À la première étape, le premier matériau absorbant (33) est disposé dans les premières régions (33A-33D, 33P-33R, 33U-33W ; 33M, 33N) alignées dans une direction où la première feuille (32A-32C) continue, dans une pluralité de rangées avec un espacement entre celles-ci, et
À la deuxième étape, le second matériau absorbant (23) est disposé dans les secondes régions (23A, 23B ; 23U-23W) alignées dans une direction où la deuxième feuille (22) continue, avec un espacement entre les deux.

2. La méthode de fabrication du corps absorbant selon l'affirmation 1, où, à la troisième étape, le corps continu (14, 14a, 14b, 14c) est formé, dans lequel la première feuille absorbante (30, 50) et la seconde feuille absorbante (20, 20a) se chevauchent de sorte que les premières régions (33a-33d, 33p-33r, 33u-33w ; 33m, 33n) et les secondes régions (23a, 23b ; 23u-23w) sont adjacents en vue de plan.

3. La méthode de fabrication du corps absorbant selon la revendication 1, comprenant en outre :
une cinquième étape de formation des premières pièces (31) incluant les premières régions (33A-33D, 33P-33R, 33U-33W ; 33M, 33N), en coupant la première feuille absorbante (30) après la première étape et avant la troisième étape ; et
une sixième étape consiste à former les secondes pièces (21) incluant la deuxième région (23A, 23B ; 23U-23W), en coupant la deuxième feuille absorbante (20) après la deuxième étape et avant la troisième étape,
où, à la troisième étape, le corps continu (14b) se forme dans lequel les premières pièces (31) et les secondes pièces (21) sont alignées de sorte que les premières régions (33a-33c) et les secondes régions (23a, 23b) soient adjacentes en vue en plan.

4. La méthode de fabrication du corps absorbant selon l'une des revendications 1 à 3, où, à la première étape, le premier matériau absorbant (33) est disposé sur plus d'une des premières feuilles (32a-32c ; 52a-52c), et
À la troisième étape, le corps continu (14, 14a, 14b, 14c) se forme dans lequel plusieurs des premières feuilles (32a-32c ; 52a-52c) sont disposées avec un espacement entre elles.

5. La méthode de fabrication du corps absorbant selon l'une des revendications 1 à 4, où, à la première étape, une première feuille de revêtement (34, 54) est reliée à la première feuille (32A-32C, 52A-52C) pour former la première feuille absorbante (30, 50) disposant le premier matériau absorbant (33) entre la première feuille (32A-32C, 52A-52C) et la première feuille de revêtement (34, 54).

6. La méthode de fabrication du corps absorbant selon l'une des revendications 1 à 5, où, à la deuxième étape, une deuxième feuille de revêtement (24) est reliée à la seconde feuille (22) pour former la deuxième feuille absorbante (20), le second matériau absorbant (23) étant disposé entre la deuxième feuille (22) et la seconde feuille de revêtement (24).
